(19) Europäisches Patentamt
European Patent Office
Office européen des brevets

(11) Veröffentlichungsnummer: **0 035 218**
**B1**

(12) # EUROPÄISCHE PATENTSCHRIFT

(45) Veröffentlichungstag der Patentschrift: **11.01.84**

(51) Int. Cl.³: **C 07 D 311/78,** C 09 B 3/18

(21) Anmeldenummer: **81101315.0**

(22) Anmeldetag: **24.02.81**

(54) **Perylen-3,4,9,10-tetracarbonsäure-monoanhydrid-monoalkalisalze, Verfahren zu ihrer Herstellung und ihre Verwendung.**

(30) Priorität: **05.03.80 DE 3008420**

(43) Veröffentlichungstag der Anmeldung: **09.09.81 Patentblatt 81/36**

(45) Bekanntmachung des Hinweises auf die Patenterteilung: **11.01.84 Patentblatt 84/2**

(84) Benannte Vertragsstaaten: **CH DE FR GB LI**

(56) Entgegenhaltungen:
**CH - A - 103 431**
**CH - A - 105 854**
**DE - B - 1 806 403**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Spietschka, Ernst, Dr., Kirchweg 3, D-6270 Idstein/Taunus (DE)**
Erfinder: **Tröster, Helmut, Dr., Am Erdbeerstein 44, D-6240 Königstein/Taunus (DE)**

**EP 0 035 218 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Perylen-3,4,9,10-tetracarbonsäuremonoanhydrid-monoalkalisalze, Verfahren zu ihrer Herstellung und ihre Verwendung

Gegenstand der vorliegenden Erfindung sind Perylen-3,4,9,10-tetracarbonsäure-monoanhydrid-monoalkalisalze der Formel 1

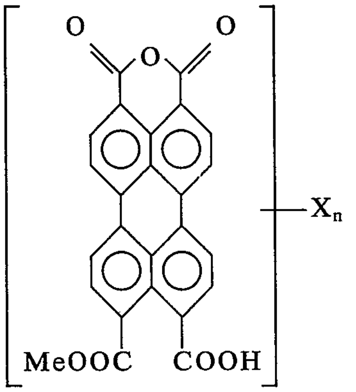 (1)

in der Me ein Natrium- oder Kalium-Ion, X Chlor oder Brom und n eine Zahl von 0-4 bedeuten. Besonders bevorzugt sind diejenigen Verbindungen der Formel 1, in denen Me für ein Kaliumatom steht und solche, in denen n Null ist.

Die Erfindung betrifft weiterhin ein Verfahren zur Herstellung der Salze der Formel 1, das dadurch gekennzeichnet ist, daß man Perylen-3,4,9,10-tetracarbonsäuresalze der Formel 2

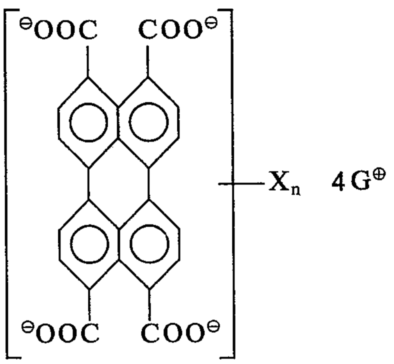

in der $G^+$ ein Kation bedeutet und X und n die obengenannte Bedeutung haben, vorzugsweise ein Tetraalkali- oder Tetraammoniumsalz, in wäßriger Lösung oder Suspension, falls erforderlich in Gegenwart von mindestens einem Äquivalent $Me^+$, wobei Me die obengenannte Bedeutung hat, bei 20 bis 100° C, vorzugsweise 70 bis 95° C, mit drei Äquivalenten Säure behandelt.

Dabei verfährt man zweckmäßig so, daß man die Perylentetracarbonsäure bzw. ihr Anhydrid in Wasser mit der erforderlichen Menge an Base in üblicher Weise bei erhöhter Temperatur in das Tetracarboxylat überführt, wobei je nach Verdünnungsgrad eine Suspension oder Lösung erhalten wird. Wurde als Base ein zur Überführung in das Tetracarboxylat geeignetes Amin verwendet, so muß mindestens das zur Monoalkalisalzbildung erforderliche Kationäquivalent in Form des Hydroxids oder eines entsprechenden Salzes zugesetzt werden.

Nach Zurücknahme eines eventuellen Basenüberschusses mit Säure werden 3 Äquivalente Säure pro Mol Perylentetracarbonsäure bei 20° —100° C, vorzugsweise bei 70—95° C zugetropft. Erfolgt die Säurezugabe ohne Wärmezufuhr, so ist es zweckmäßig, die Reaktionsmischung anschließend zur Überführung in das Monoanhydrid zu erwärmen. Das Reaktionsprodukt kann jedoch auch ohne vorheriges Erwärmen isoliert werden. Bei der Trocknung geht es in das Monoanhydrid über.

Zur Erzielung einer hohen Produktreinheit ist es günstig, den Säurezulauf so zu regulieren, daß der pH-Wert dabei nicht unter 3 absinkt; vorzugsweise stellt man einen weitgehend konstanten Bereich von 4—7 ein.

Der Säurebedarf richtet sich nach dem Endpunkt der Protonierung, der durch einen pH-Sprung auf einen pH-Bereich von 3,5—6,5 gekennzeichnet ist.

Das ausgefallene schwer lösliche Perylen-3,4,9,10-tetracarbonsäuremonoanhydridmonoalkalisalz kann in üblicher Weise durch Filtration isoliert werden. Es kann jedoch auch ohne Zwischenisolierung weiter umgesetzt werden.

Zum Lösen der Perylen-3,4,9,10-tetracarbonsäure kommen als Basen insbesondere die Hydroxide und Carbonate des Natriums und Kaliums in Betracht. Geeignete Amine sind sekundäre und tertiäre Amine, deren Basizität ausreicht, um die Perylentetracarbonsäure in ihr Tetraammoniumsalz zu überführen, wie beispielsweise Dimethylamin, Dibutylamin, Trimethylamin, Triethylamin, Diethanolamin oder Triethanolamin.

Geeignete Alkaliionenspender sind Hydroxide, Chloride, Sulfate, Nitrate oder auch Carbonate des Natriums und insbesondere des Kaliums.

Als Säuren verwendet man zweckmäßig starke Mineralsäuren, wie beispielsweise Salz-, Schwefel-, Salpeter- und Phosphorsäure. Es können auch saure Salze wie Natrium- oder Kaliumhydrogensulfat sowie organische Säuren wie Essigsäure, Propionsäure, Trichloressigsäure oder Toluolsulfonsäure Verwendung finden.

Die neuen Verbindungen stellen wertvolle Ausgangsprodukte zur Herstellung von Farbstoffen und Pigmenten, insbesondere zur Herstellung von asymmetrisch N-substituierten Perylentetracarbonsäurediimidpigmenten dar.

Gegenstand der Erfindung ist deshalb auch die Verwendung der neuen Verbindungen zur Herstellung von Farbmitteln.

Aus CH-A-105 854 ist es bekannt, daß sich Perylentetracarbonsäure durch Lösen in überschüssigem Alkali in ihre löslichen (Tetra)alkalisalze überführen läßt. Es geht ferner noch daraus hervor, daß die Alkalisalze der chlorierten Perylentetracarbonsäure leichter löslich sind als die der chlorfreien Perylentetracarbonsäure.

Das in DE-B-1 806 403 beschriebene Verfahren unterscheidet sich grundlegend vom erfindungsgemäßen Verfahren sowohl bezüglich der

Endprodukte als auch in den Herstellungsbedingungen. Das bekannte Verfahren stellt einen Konditionierungsprozeß dar, der darauf abzielt, durch Zugabe von überschüssigen Säuren zu Perylentetracarboxylatlösungen in Gegenwart eines Dispergators Perylentetracarbonsäuredianhydrid in einer Pigmentform mit verbesserten anwendungstechnischen Eigenschaften zu erhalten (vgl. darin etwa Beispiel 1, S. 7, Zeile 12 von unten ff.). Von der Herstellung von Mono-natrium- bzw. kaliumsalzen des Perylentetracarbonsäure-monoanhydrids wird auch in dieser Entgegenhaltung nichts ausgesagt.

In den folgenden Beispielen beziehen sich Prozentangaben auf das Gewicht, wenn nichts anderes angegeben ist.

Beispiel 1

19,6 g Perylen-3,4,9,10-tetracarbonsäuredianhydrid und 8,3 g Natriumhydroxid (100%ig) werden in 2500 ml Wasser bei 80° C in Lösung gebracht. Anschließend wird im Verlauf von etwa 2 Stunden bei dieser Temperatur durch Zutropfen von 61 g 10%iger Salzsäure der pH-Wert (Einstabmeßkette) auf 4,5 eingestellt. Es wird 2 Stunden bei 80° C nachgerührt. In dieser Zeit steigt der pH-Wert der Suspension nur noch geringfügig auf etwa 5,0. Das Reaktionsprodukt wird bei 20—25° C abgesaugt, mit Wasser chloridfrei gewaschen und getrocknet.

Ausbeute: 21,3 g
Analyse: ber. Na. 5,3%
gef. Na 3,5% ≙ 65,7% d. Th.

Beispiel 2

196 g Perylen-3,4,9,10-tetracarbonsäuredianhydrid werden in 2240 g 5%iger Kalilauge bei 90° C gelöst. Die erhaltene Lösung hat einen pH-Wert von etwa 10,5 (Einstabmeßkette). Bei 90° C werden im Verlauf von 2—3 Stunden 1432 g 10%ige Phosphorsäure zugetropft. Nach Beendigung der Säurezugabe ist der pH-Wert auf 5,0 gefallen. Es wird noch 1 Stunde bei gleicher Temperatur nachgerührt. Der pH-Wert der Suspension bleibt hierbei praktisch unverändert. Das ausgefallene bordofarbene Kaliumsalz wird bei 20—25° C abgesaugt, mit Wasser phosphatfrei gewaschen und bei 110° C getrocknet.

Ausbeute: 220 g
Analyse: ber. C 64,3% ; H 2,0%; K 8,7%
gef. C 63,7%; H 2,0%; K 8,6%

Anstelle von 10%iger Phosphorsäure kann mit gleichem Erfolg auch die äquivalente Menge (168 g) 85%ige Phosphorsäure eingesetzt werden.

Beispiel 3

Eine Mischung aus 196 g Perylen-3,4,9,10-tetracarbonsäuredianhydrid, 2000 ml Wasser und 132,9 g 85%igem Kaliumhydroxid wird auf 90° C erwärmt, wobei sich ein pH-Wert von 10—11 einstellt. Im Verlauf von etwa 2 Stunden wird bei dieser Temperatur durch Zutropfen von 180 g Salzsäure (31%ig) ein praktisch konstanter pH-Wert von 4,5—5,0 eingestellt. Nach einer Nachrüstzeit von 1 Stunde wird das ausgefallene Kaliumsalz heiß abgesaugt und mit heißem Wasser chlorionenfrei gewaschen. Nach dem Trocknen erhält man 219 g dunkelrotes Produkt, das mit dem Reaktionsprodukt aus Beispiel 2 identisch ist.

Beispiel 4

In eine Lösung von 27,8 g Triethylamin in 500 ml Wasser werden 19,6 g Perylen-3,4,9,10-tetracarbonsäuredianhydrid eingetragen und durch Erwärmen auf 80° C in Lösung gebracht. Zur klaren Lösung werden 3,8 g Kaliumchlorid zugegeben und anschließend bei dieser Temperatur in 2—3 Stunden durch Zutropfen von 185 ml 1 N Salzsäure ein nahezu konstanter pH-Wert von 4—5 eingestellt. Eine Tüpfelprobe auf Filterpapier zeigt einen praktisch farblosen Auslauf. Nach einer Nachrührzeit von etwa 2 Stunden bei 80° C wird das dunkelrote mikrokristallin ausgefallene Kaliumsalz in üblicher Weise isoliert.

Ausbeute: 21,9 g
Analyse: K: 7,6% ≙ 87,3% d. Th.

Beispiel 5

19,6 g Perylen-3,4,9,10-tetracarbonsäuredianhydrid werden in 2500 ml Wasser suspendiert und durch Zugabe von 8,0 g Natriumhydroxid bei 80° C gelöst. Nach Zusatz von 3,8 g Kaliumchlorid wird bei pH-Wert mit 1 N Salzsäure auf 7,5 eingestellt und anschließend in etwa 2 Stunden bei 80° C durch Zutropfen von 150 ml 1 N Salzsäure auf einen nahezu konstanten pH-Wert von 4,5 eingestellt. Nach 1 Stunde Nachrührzeit wird das in bordofarbenen Nadeln ausgefallene Reaktionsprodukt isoliert.

Ausbeute: 21,5 g
Analyse: K: 6,7%; Na 0,55% ≙ 87,3% d. Th. (bez. auf Monoalkalisalz).

Beispiel 6

Zu 550 g einer wäßrigen Lösung, die 0,1 Mol des Tetrakaliumsalzes der Perylen-3,4,9,10-tetracarbonsäure enthält, werden bei 90° C 38,6 g

50%ige Essigsäure langsam bis zu einem nahezu konstanten pH-Wert von 6,2 zugetropft. Nach 1,5 Stunden Nachrührzeit bei 90°C wird heiß abgesaugt und mit heißem Wasser gewaschen. Man erhält 43,8 g Monokaliumsalz. Das Produkt entspricht dem gemäß Beispiel 2 erhaltenen.

Beispiel 7

Zu 2600 ml einer wäßrigen Lösung mit einem Gehalt von 0,5 Mol des Tetrakaliumsalzes der Perylentetracarbonsäure werden bei 20—25°C in 30 Minuten 547,5 g 10%ige Salzsäure zugetropft. Anschließend erwärmt man auf 90°C und hält 30 Minuten bei dieser Temperatur. Das Reaktionsprodukt entspricht dem in Beispiel 2 beschriebenen.

Ausbeute: 218 g

Beispiel 8

Zu 606,3 g einer 0,1-molaren Lösung des Tetrakaliumsalzes der Perylentetracarbonsäure werden bei 20—25°C in etwa 3 Stunden 109 g 10%ige Salzsäure zugetropft, wobei der pH-Wert der Lösung bei 6—7 liegt. Mit 1 g 10%iger Salzsäure wird der pH-Wert auf 5,0—5,5 eingestellt. Nach einigen Stunden wird das Reaktionsprodukt wie üblich isoliert und getrocknet.

Ausbeute: 42,5 g
Analyse: K: 7,8% ≙ 89,6% d. Th.

Beispiel 9

23,6 g Bromperylentetracarbonsäuredianhydrid (Bromgehalt: 14,4% ≙ 0,82 Br-Atome) werden in 500 ml Wasser durch Zugabe von 13,2 g Kaliumhydroxid (88%ig) bei 80°C gelöst. Durch Zutropfen von 56 g 10%iger Salzsäure wird bei dieser Temperatur ein pH-Wert von 4—5 eingestellt und das gebildete Kaliumsalz nach 1 Stunde isoliert.

Ausbeute: 25,7 g
Analyse: K: 6,6%; Br: 12,8%

Das Bromperylentetracarbonsäuredianhydrid wurde gemäß Beispiel 1 der DE-OS 2 519 790 hergestellt, wobei anstelle von Chlor die äquivalente Menge Brom eingesetzt wurde.

Beispiel 10

26,5 Tetrachlorperylentetracarbonsäuredianhydrid (Chlorgehalt: 27,2%ig) hergestellt gemäß Beispiel 3 der DE-OS 2 519 790, werden bei 80°C in 500 ml Wasser und 13,2 g Kaliumhydroxid (85%ig) gelöst. Bei dieser Temperatur wird durch Zutropfen von 57 g 10%iger Salzsäure der pH auf 3—4 eingestellt und das ausgefallene Reaktionsprodukt aus der heißen Lösung nach einer Nachrührzeit von 1 Stunde isoliert. Man erhält 25,7 g des entsprechenden Monoanhydrid-monokaliumsalzes.

Analyse: Cl: 24,5%
K: 6,3%

**Patentansprüche**

1. Perylen-3,4,9,10-tetracarbonsäure-monoanhydrid-monoalkalisalze der Formel 1

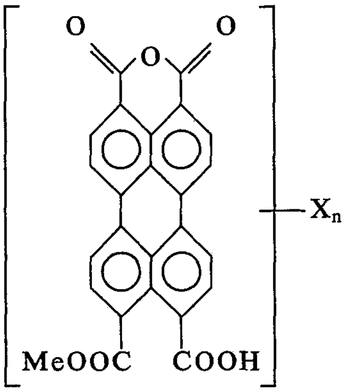 (1)

in der Me für ein Natrium- oder Kalium-Ion steht, X Chlor oder Brom ist und n eine Zahl von 0 bis 4 bedeutet.

2. Verbindung gemäß Anspruch 1, dadurch gekennzeichnet, daß n = 0 ist.

3. Verbindung gemäß Anspruch 1, dadurch gekennzeichnet, daß Me ein Kaliumion bedeutet.

4. Die Verbindung nach Anspruch 1, in der n Null und Me Kalium ist.

5. Verfahren zur Herstellung der Verbindungen gemäß Anspruch 1, dadurch gekennzeichnet, daß man Perylen-3,4,9,10-tetracarbonsäuresalze der Formel 2

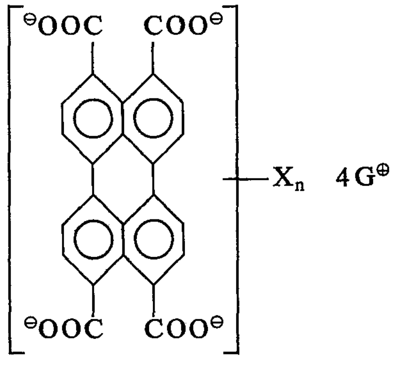 (2)

in der $G^+$ ein Kation bedeutet, falls erforderlich in Gegenwart von mindestens 1 Äquivalent $Me^+$, bei 20 bis 100°C mit 3 Äquivalenten Säure behandelt, wobei Me, X und n die im Anspruch 1 genannte Bedeutung haben.

6. Verfahren nach Anspruch 5, dadurch gekennzeichnet, daß man die Säurebehandlung bei 70—95°C durchführt.

7. Verfahren nach Anspruch 5 und 6, dadurch gekennzeichnet, daß Me Kalium bedeutet und n = 0 ist.

8. Verfahren nach Anspruch 5 bis 7, dadurch gekennzeichnet, daß man einen weitgehend konstanten pH-Bereich von 4—7 einstellt.

9. Verwendung der Verbindungen gemäß Anspruch 1—4 zur Herstellung von Farbmitteln.

## Claims

1. Monoalkali metal salts of perylene-3,4,9,10-tetracarboxylic acid monoanhydride of the formula 1

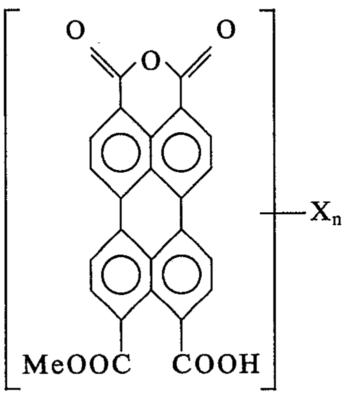

(1)

wherein Me is sodium or potassium, X is chlorine or bromine and n is a number of from zero to 4.

2. Compounds as claimed in claim 1, wherein n is zero.

3. Compounds as claimed in claim 1, wherein Me is potassium.

4. The compound as claimed in claim 1, wherein n is zero and Me is potassium.

5. A process for preparing compounds as claimed in claim 1, which comprises reacting perylene-3,4,9,10-tetracarboxylic acid salts of the formula 2

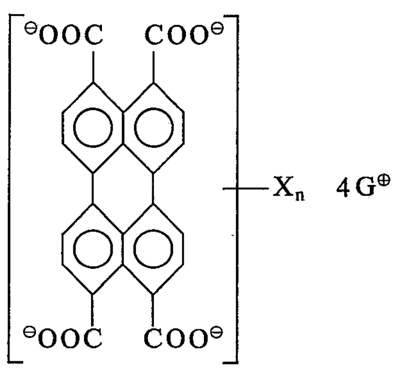

(2)

wherein X and n are defined as in claim 1 and G+ is a cation, if necessary in the presence of at least 1 molar equivalent Me+, wherein Me is defined as in claim 1, at a temperature of 20 to 100°C with 3 molar equivalents of a sufficiently strong acid.

6. A process as claimed in claim 5, wherein the temperature is from 70 to 95°C.

7. A process as claimed in claims 5 and 6, wherein Me is potassium and n is zero.

8. A process as claimed in claims 5—7, wherein the reaction is performed in a pH-range of 4 to 7.

9. Use of the compounds as claimed in claims 1—4 for the manufacture of dyestuffs.

## Revendications

1. Sels monoalcalins du monoanhydride de l'acide pérylène-3,4,9,10-tétracarboxylique de formule 1 ci-dessous:

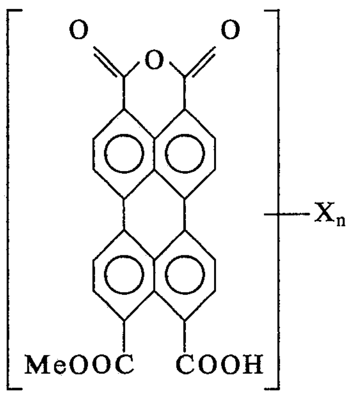

(1)

dans laquelle Me représente un ion sodium ou potassium, X le chlore ou le brome et n est un nombre de 0 à 4.

2. Composés selon la revendication 1, dans lesquels n = 0.

3. Composés selon la revendication 1, dans lesquels me est l'ion potassium.

4. Composé selon la revendication 1, dans lequel n est nul et Me est l'ion potassium.

5. Procédé de préparation des composés selon la revendication 1, caractérisé en ce que l'on traite avec trois équivalents d'un acide, entre 20 et 100°C, des sels de l'acide pérylène-3,4,9,10-tétracarboxylique de formule 2

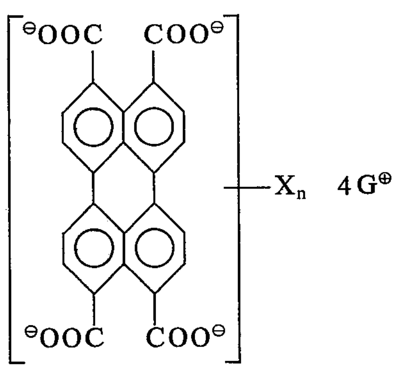

(2)

G+ désignant un cation, si cela est nécessaire en présence d'un ou de plus d'un équivalent d'un cation Me+, Me, X et n ayant les significations données à la revendication 1.

6. Procédé selon la revendication 5, caractérisé en ce que le traitement avec l'acide se fait entre 70 et 95°C.

7. Procédé selon la revendication 5 ou 6, caractérisé en ce que Me est le potassium et n = 0.

8. Procédé selon l'une quelconque des revendications 5 à 7, caractérisé en ce que l'on ajuste le mélange réactionnel à un pH à peu près constant, compris entre 4 et 7.

9. Emploi des composés selon l'une quelconque des revendications 1 à 4 pour la fabrication de matières colorantes.